# EUROPEAN PATENT APPLICATION

(11) **EP 3 400 929 A1**
(43) Date of publication of application: **14.11.2018**
(21) Application number: 17185285.8
(22) Date of filing: 08.08.2017
(51) Int. Cl.: A61K 6/02, A61C 13/00, C04B 35/00

(54) **DENTAL ZIRCONIA RESTORATION MATERIAL WITH UNIFORM TRANSITION OF STRENGTH AND COLOR, AND PREPARATION METHOD THEREOF**

(30) Priority: 12.05.2017 CN 201710334979
(71) Applicant: Aidite (Qinhuangdao) Technology Co.Ltd., Qinhuangdao City 066004 (CN)
(72) Inventor: LI, Hongwen, Qinhuangdao City, 066004 (CN); WU, Haiyan, Qinhuangdao City, 066004 (CN); CHEN, Yingying, Qinhuangdao City, 066004 (CN)
(74) Representative: Markieta, Jaroslaw Franciszek

(57) **Abstract**

The application discloses a dental zirconia restoration material with uniform transition of strength and color, and a preparation method thereof. The preparation method includes the following steps: (1) pouring colored zirconia powder into a dry pressing mould in accordance with a sequence of the strength from high to low and the color from dark to light for each layer, and performing dry pressing; (2) performing isostatic cool pressing after the dry pressing; (3) performing pre-sintering after the isostatic cool pressing to obtain a greenware; and (4) performing CAD/CAM cutting on the greenware, and finally performing final sintering to obtain the dental zirconia restoration material. In the present application, the strength of a restoration can gradually increase from a cut end to the neck, the wear to adjacent teeth and jaw teeth is reduced, and long-bridge restoration can be realized due to the high strength of the neck.

## Description

The present disclosure generally relates to the field of dentistry, and in particular to a dental zirconia restoration material with uniform transition of strength and color, and a preparation method thereof.

Ceramic materials have become one of main materials for substituting natural dental hard tissues by virtue of excellent simulative aesthetic effects and biocompatibility, wherein zirconia ceramic is the most popular choice at present. With the improvement of preparation processes of dental zirconia material, its transparency, color and other properties have satisfy the aesthetic needs, however, due to the very large differences between the basic mechanical parameters of zirconia and the natural teeth, especially the hardness and elastic modulus of zirconia are significantly greater than those of dental enamel, zirconia ceramic restorations and the natural teeth generate frictional wear in chewing movement processes, and excessive wear may cause damage to the natural teeth. The wear of the natural teeth is a natural process, but the excessive wear of the natural teeth may cause damage to the organs of the body itself. Therefore, how to reduce the wear of the zirconia restorations to the natural teeth in using processes is a problem which should be solved urgently in clinical applications.

Chinese Patent Application Publication No. CN105622096A, entitled "Gradient-Color Zirconia Material And Preparation Method Thereof" relates to a zirconia material, which includes a three-layer structure consisting of a bottom layer, an intermediate layer and an outer layer, and the preparation method includes: respectively mixing nano zirconia powder, iron oxide and erbium oxide according to different proportions, then placing the compositions at different positions of a die cavity, and performing dry pressing, cold isostatic pressing, pre-sintering, cutting and secondary sintering to obtain the color gradient zirconia material.

In the Chinese Patent Application Publication No. CN104844200A, entitled "Multiple-oxide-doped gradient-color zirconia dental restoration and preparation method thereof", the gradient color zirconia dental restoration is prepared by mixing more than one of the three kinds of dental zirconia ceramic mixed powder ABC with powder D on each ceramic layer according to different mass fractions and overlaying, wherein the three kinds of mixed powder are respectively ErO₂, CeO₂ and Fe₂O₃ doped zirconia ceramic powder raw materials, and the powder D is an undoped ceramic powder raw material.

The Application Publication No. CN105584161A, entitled "Transparent gradient color zirconia material and preparation process thereof", relates to a transparent gradient color zirconia material and a preparation process thereof, and belongs to the technical field of dental ceramic materials. The transparent gradient color zirconia material includes a five-layer structure consisting of a bottom layer, an intermediate layer A, an intermediate layer B, an intermediate layer C and an upper layer, which are arranged in sequence from bottom to top, which are respectively prepared from three powers of 3Y-TZP, (3.5-4.5)Y-PSZ and (4.5-6)Y-PSZ powder and a coloring agent according to different mass ratios.
In the prior art, the color and transparency of zirconia are basically studied. Although the aesthetic properties of the dental zirconia restoration materials are improved, the wear of the dental zirconia restorations to adjacent teeth and jaw teeth in chewing movement processes is neglected.

In view of the above defects or shortcomings in the prior art, the present application aims at providing a dental zirconia restoration material with uniform transition of strength and color, which can reduce the damage to adjacent teeth and jaw teeth in chewing movement processes, and a preparation method thereof.
To achieve the above purpose, the present application adopts the following technical solutions:
A preparation method of a dental zirconia restoration material with uniform transition of strength and color comprises the following steps:
   (1) pouring colored zirconia powder into a dry pressing mould in accordance with a sequence of the strength from high to low and the color from dark to light for each layer, and performing dry pressing;
   (2) performing isostatic cool pressing after the dry pressing;
   (3) performing pre-sintering after the isostatic cool pressing to obtain a greenware; and
   (4) performing CAD/CAM (Computer Aided Design/Computer Aided Manufacturing) cutting on the greenware, and finally performing final sintering to obtain the dental zirconia restoration material.

The present application further provides a dental zirconia restoration material with uniform transition of strength and color prepared according to the above preparation method. Compared with the prior art, the present application has the following beneficial effects:
In the present application, multiple kinds of colored zirconia powder with different strength are delaminated and pressed to prepare the dental zirconia restoration material with uniform transition of strength and color, so the strength of a restoration can gradually increase from a cut end to the neck, the wear to the adjacent teeth and the jaw teeth is reduced, and long-bridge restoration can be realized due to the high strength of the neck. In addition, the color gradually becomes darker from the cut end to the neck, uniform transition of the color is realized, the color of the restoration is closer to that of the natural teeth, and a specific color blending method satisfying the colors of VITA 16-color shade guides of the international colorimetric standard is particularly illustrated in the present application.

The present application will be described in further detail in combination with embodiments. It can be understood that the specific embodiments described herein are only for the purpose of explaining the related invention rather than limiting the present application.
It should be noted that the embodiments and the features in the embodiments in the present application may be combined with each other in case of no conflict.
The present application provides a preparation method of a dental zirconia restoration material with uniform transition of strength and color, comprises the following steps:
(1) pouring colored zirconia powder into a dry pressing mould in accordance with a sequence of the strength from high to low and the color from dark to light for each layer, and performing dry pressing;
(2) performing isostatic cool pressing after the dry pressing;
(3) performing pre-sintering after the isostatic cool pressing to obtain a greenware; and
(4) performing CAD/CAM cutting on the greenware, and finally performing final sintering to obtain the dental zirconia restoration material.

According to the present application, the clinical requirements for the colors of the zirconia restorations can be met, and the strength gradually reduces from the cut end to the neck, so that the damage to adjacent teeth and jaw teeth in chewing movement processes can be greatly reduced, and thus zirconia restorations better match natural teeth on mechanical properties and colors.

Preferably, the dental zirconia restoration material includes 6-8 ceramic layers.
The larger the number of layers is, the smaller the color difference between two adjacent layers is, the more natural the transition of color of the restoration is; however, the larger the layer number is, the higher the requirements for the production technology is, so the present application selects a layer range of 6-8 layers after comprehensively considering the factors in the two aspects, thereby not only ensuring natural transition of color, but also being conducive to the production.

Further, when a first ceramic layer is a cut end, the strength of the colored zircon ia powder of the first ceramic layer is 300-800 MPa; the difference of the strength of the colored zirconia powder of two adjacent ceramic layers is 20-200 MPa; and the strength of the dental zirconia restoration material gradually increases from the cut end to the neck, and the color gradually becomes darker from the cut end to the neck.

In the present application, the strength range of each ceramic layer can be properly adjusted according to the wear condition of the teeth in the mouth of a patient, as the strengths of the enamel and the dentin of the natural teeth are not very high, so the strength of the cut end of the restoration does not need to be too high in order to match with the natural teeth, the strength of the cut end can be properly increased while considering that a very large occlusal force may be needed to be borne sometimes, therefore the reasonable strength design of each ceramic layer enables the restoration to better simulate the natural teeth, and the functions are also matched.

The preparation method in the present application is simple and easy, and the strength distribution characteristics of the prepared dental zirconia restoration greatly reduce the wear of the restoration to the adjacent teeth and the jaw teeth.

The weight percent content of the colored zirconia powder of the first ceramic layer is 15-25%, the difference of the weight percent contents of the colored zirconia powders of two adjacent ceramic layers at the middle is 0±2%, and the weight percent content of the colored zirconia powder of the last ceramic layer is 18-55%.

The two adjacent ceramic layers at the middle refers to intermediate ceramic layers between the first ceramic layer and the last ceramic layers, the contents of the colored zirconia powder of each adjacent ceramic layers are different in the intermediate ceramic layers, and a mass percent difference of 0±2% is present.

In the present application, the first ceramic layer is the cut end and is located at the top end, the strength of the top end is the lowest, so the wear of the restoration to the adjacent teeth and the jaw teeth can be greatly reduced, and the thickness of the first layer is properly increased, so the color effect of the restoration is more natural. The last layer is a substrate layer and is located at the neck, its strength is the highest, and thus the restoration can bear a larger occlusal force in a chewing process without breaking and damaging the neck edge part. The thickness of the substrate layer may be increased to further prolong the fatigue life of the restoration; as for a long-bridge restoration with larger span, the larger the thickness of the substrate layer is, the longer the fatigue life of the restoration is.

Further, the colored zirconia powder of each strength contains A, B, C and D color systems satisfying the colors of a VITA 16-color shade guide of the international colorimetric standard, wherein the color range of the A color system is A0.1 -A4, the color range of the B color system is B0.1-B4, the color range of the C color system is C0.1-C4, and the color range of the D color system is D0.1-D4; and after the color system of one ceramic layer is determined, the color system is selected for the rest ceramic layers, and the rule is followed that the color gradually becomes darker from the cut end to the neck.

Referring to Table 1, in the present application, the color powder of the A color system and the white zirconia powder are mixed into the powders with different colors in the same color system according to different mass fraction ratios, the shades are distinguished according to the adding proportion of the powder, for example, if the adding weight percent content of the color powder of the A color system is 1%, and the adding weight percent content of the white zirconia powder is 99%, the color is marked as A0.1; if the adding weight percent content of the color powder of the A color system is 10%, and the adding weight percent content of the white zirconia powder is 90%, the color is marked as A1; if the adding proportion of the color powder of the A color system is 40%, the color is marked as A4; and if the adding weight percent content of the color powder of the A color system is 15%, the color is marked as A1.5.

Similarly, the color powder of the A color system is replaced with the color powder of the B color system, the mass fraction of the added color powder of the B color system is changed to obtain a series of color powder of the B color system, and different colors of the same color system B0.1-B4 can be obtained.

Similarly, the color powder of the A color system is replaced with the color powder of the C color system, the mass fraction of the added color powder of the C color system is changed to obtain a series of color powder of the C color system, and different colors of the same color system C0.1-C4 can be obtained.

Similarly, the color powder of the A color system is replaced with the color powder of the D color system, the mass fraction of the added color powder of the D color system is changed to obtain a series of color powder of the D color system, and different colors of the same color system D0.1-D4 can be obtained.

Preferably, the dry pressing pressure is 3-15MPa, and the dry pressing time is 5-30s. Preferably, the isostatic cool pressing pressure is 80-270 MPa, and the pressure is maintained for 60-200s.

Preferably, the pre-sintering temperature is 800-1100 °C, and the pre-sintering time is 90-180 min.

Preferably, the final sintering temperature is 1450-1550 °C, and the final sintering time is 60-180 min.

By selecting the above temperatures and pressures in the present application, the density of the restoration can be improved, the cutting performance is improved, the transparency of the finally sintered restoration is high, and the color thereof is natural.

The present application further provides a dental zirconia restoration material with uniform transition of strength and color prepared by the above preparation method.
The present application will be further illustrated below through specific embodiments:
Embodiment 1
   A preparation method of a dental zirconia restoration material with uniform transition of strength and color comprises the following steps:
   (1) pouring colored zirconia powder into a dry pressing mould in accordance with a sequence of the strength from high to low and the color from dark to light for each layer, and performing dry pressing, wherein the dry pressing pressure is 10 MPa, and the dry pressing time is 15s;
   (2) performing isostatic cool pressing after the dry pressing, wherein the isostatic cool pressing pressure is 180 MPa, and the pressure is maintained for 150s;
   (3) performing pre-sintering after the isostatic cool pressing to obtain a greenware, wherein the pre-sintering temperature is 1000 °C, and the pre-sintering time is 150 min; and
   (4) performing CAD/CAM cutting on the greenware, and finally performing final sintering to obtain the dental zirconia restoration material, wherein the final sintering temperature is 1530 °C, and the final sintering time is 120 min.
Embodiment 2
   A preparation method of a dental zirconia restoration material with uniform transition of strength and color comprises the following steps:
   (1) pouring colored zirconia powder into a dry pressing mould in accordance with a sequence of the strength from high to low and the color from dark to light for each layer, and performing dry pressing, wherein the dry pressing pressure is 15 MPa, and the dry pressing time is 5s;
   (2) performing isostatic cool pressing after the dry pressing, wherein the isostatic cool pressing pressure is 270 MPa, and the pressure is maintained for 60s;
   (3) performing pre-sintering after the isostatic cool pressing to obtain a greenware, wherein the pre-sintering temperature is 800 °C, and the pre-sintering time is 180 min; and
   (4) performing CAD/CAM cutting on the greenware, and finally performing final sintering to obtain the dental zirconia restoration material, wherein the final sintering temperature is 1450 °C, and the final sintering time is 60 min.
Embodiment 3
   A preparation method of a dental zirconia restoration material with uniform transition of strength and color comprises the following steps:
   (1) pouring colored zirconia powder into a dry pressing mould in accordance with a sequence of the strength from high to low and the color from dark to light for each layer, and performing dry pressing, wherein the dry pressing pressure is 3 MPa, and the dry pressing time is 30s;
   (2) performing isostatic cool pressing after the dry pressing, wherein the isostatic cool pressing pressure is 120 MPa, and the pressure is maintained for 200s;
   (3) performing pre-sintering after the isostatic cool pressing to obtain a greenware, wherein the pre-sintering temperature is 1100 °C, and the pre-sintering time is 60 min; and
   (4) performing CAD/CAM cutting on the greenware, and finally performing final sintering to obtain the dental zirconia restoration material, wherein the final sintering temperature is 1550 °C, and the final sintering time is 180 min.

**Table 1 color mark**

| Color mark | Weight percent content of the added color powder | Weight percent content of the white zirconia powder Added |
|---|---|---|
| A0.1 | Color powder of the A color system 1% | 99% |
| A0.3 | Color powder of the A color system 3% | 97% |
| A0.5 | Color powder of the A color system 5% | 95% |
| A0.6 | Color powder of the A color system 6% | 94% |
| A0.8 | Color powder of the A color system 8% | 92% |
| A1 | Color powder of the A color system 10% | 90% |
| A1.5 | Color powder of the A color system 15% | 85% |
| A2 | Color powder of the A color system 20% | 80% |
| A2.5 | Color powder of the A color system 25% | 75% |
| A3 | Color powder of the A color system 30% | 70% |
| A3.5 | Color powder of the A color system 35% | 65% |
| A4 | Color powder of the A color system 40% | 60% |
| B0.1 | Color powder of the B color system 1% | 99% |
| B0.3 | Color powder of the B color system 3% | 97% |
| B0.5 | Color powder of the B color system 5% | 95% |
| B0.6 | Color powder of the B color system 6% | 94% |
| B0.8 | Color powder of the B color system 8% | 92% |
| B1 | Color powder of the B color system 10% | 90% |
| B1.5 | Color powder of the B color system 15% | 85% |
| B2 | Color powder of the B color system 20% | 80% |
| B2.5 | Color powder of the B color system 25% | 75% |
| B3 | Color powder of the B color system 30% | 70% |
| B3.5 | Color powder of the B color system 35% | 65% |
| B4 | Color powder of the B color system 40% | 60% |
| C0.1 | Color powder of the C color system 1% | 99% |
| C0.3 | Color powder of the C color system 3% | 97% |
| C0.5 | Color powder of the C color system 5% | 95% |
| C0.6 | Color powder of the C color system 6% | 94% |
| C0.8 | Color powder of the C color system 8% | 92% |
| C1 | Color powder of the C color system 10% | 90% |
| C1.5 | Color powder of the C color system 15% | 85% |
| C2 | Color powder of the C color system 20% | 80% |
| C2.5 | Color powder of the C color system 25% | 75% |
| C3 | Color powder of the C color system 30% | 70% |
| C3.5 | Color powder of the C color system 35% | 65% |
| C4 | Color powder of the C color system 40% | 60% |
| D0.5 | Color powder of the D color system 5% | 95% |
| D0.6 | Color powder of the D color system 6% | 94% |
| D0.8 | Color powder of the D color system 8% | 92% |
| D1 | Color powder of the D color system 10% | 90% |
| D1.5 | Color powder of the D color system 15% | 85% |
| D2 | Color powder of the D color system 20% | 80% |
| D2.5 | Color powder of the D color system 25% | 75% |
| D3 | Color powder of the D color system 30% | 70% |
| D3.5 | Color powder of the D color system 35% | 65% |
| D4 | Color powder of the D color system 40% | 60% |

The relationship between the strength and the corresponding color, the correspondence relationship between the color and the number of layers, the correspondence relationship between the strength and the number of layers, and the correspondence relationship among the final strength, the color and the number of layers in the embodiments are shown as follows.

**Table 2 strength and corresponding color**

| Strength | Color |
|---|---|
| 300-1300MPa | A0.1, A0.3, A0.5, A0.6, A0.8, A1, A1.5, A2, A2.5, A3, A3.5, A4 B0.1, B0.3, B0.5, B0.6, B0.8, B1, B1.5, B2, B2.5, B3, B3.5, B4 C0.1, C0.3, C0.5, C0.6, C0.8, C1, C1.5, C2, C2.5, C3, C3.5, C4 D0.5, D0.6, D0.8, D1, D1.5, D2, D2.5, D3, D3.5, D4 |

**Table 3 Combination relationship between the color and the number of layers**

| Embodiment | With 6 layers as an example for the color |
|---|---|
| Embodiment 1 | A0.1, A0.3, A0.5, A0.6, A0.8, A1 |
| Embodiment 2 | A0.5, A0.6, A0.8, A1, A1.5, A2 |
| Embodiment 3 | A0.8, A1, A1.5, A2, A2.5, A3 |
| Embodiment 4 | A1, A1.5, A2, A2.5, A3, A3.5 |
| Embodiment 5 | A1.5, A2, A2.5, A3, A3.5, A4 |
| Embodiment 6 | B0.1, B0.3, B0.5, B0.6, B0.8, B1 |
| Embodiment 7 | B0.5, B0.6, B0.8, B1, B1.5, B2 |
| Embodiment 8 | B0.8, B1, B1.5, B2, B2.5, B3 |
| Embodiment 9 | B1.5, B2, B2.5, B3, B3.5, B4 |
| Embodiment 10 | C0.1, C0.3, C0.5, C0.6, C0.8, C1 |
| Embodiment 11 | C0.5, C0.6, C0.8, C1, C1.5, C2 |
| Embodiment 12 | C0.8, C1, C1.5, C2, C2.5, C3 |
| Embodiment 13 | C1.5, C2, C2.5, C3, C3.5, C4 |
| Embodiment 14 | D0.5, D0.6, D0.8, D1, D1.5, D2 |
| Embodiment 15 | D0.8, D1, D1.5, D2, D2.5, D3 |
| Embodiment 16 | D1.5, D2, D2.5, D3, D3.5, D4 |

**Table 4 Combination relationship between the color and the number of layers**

| Embodiment | The colors of 7 layers as an example |
|---|---|
| Embodiment 17 | A0.3, A0.5, A0.6, A0.8, A1, A1.5, A2 |
| Embodiment 18 | A0.6, A0.8, A1, A1.5, A2, A2.5, A3 |
| Embodiment 19 | A0.8, A1, A1.5, A2, A2.5, A3, A3.5 |
| Embodiment 20 | A1, A1.5, A2, A2.5, A3, A3.5, A4 |
| Embodiment 21 | B0.3, B0.5, B0.6, B0.8, B1, B1.5, B2 |
| Embodiment 22 | B0.6, B0.8, B1, B1.5, B2, B2.5, B3 |
| Embodiment 23 | B1, B1.5, B2, B2.5, B3, B3.5, B4 |
| Embodiment 24 | C0.1, C0.3, C0.5, C0.6, C0.8, C1 |
| Embodiment 25 | C0.3, C0.5, C0.6, C0.8, C1, C1.5, C2 |
| Embodiment 26 | C0.6, C0.8, C1, C1.5, C2, C2.5, C3 |
| Embodiment 27 | C1, C1.5, C2, C2.5, C3, C3.5, C4 |
| Embodiment 28 | D0.6, D0.8, D1, D1.5, D2, D2.5, D3 |
| Embodiment 29 | D1, D1.5, D2, D2.5, D3, D3.5, D4 |

**Table 5 Combination relationship between the color and the number of layers**

| Embodiment | The colors of 8 layers as an example |
|---|---|
| Embodiment 30 | A0.1, A0.3, A0.5, A0.6, A0.8, A1, A1.5, A2 |
| Embodiment 31 | A0.5, A0.6, A0.8, A1, A1.5, A2, A2.5, A3 |
| Embodiment 32 | A0.6, A0.8, A1, A1.5, A2, A2.5, A3, A3.5 |
| Embodiment 33 | A0.8, A1, A1.5, A2, A2.5, A3, A3.5, A4 |
| Embodiment 34 | B0.1, B0.3, B0.5, B0.6, B0.8, B1, B1.5, B2 |
| Embodiment 35 | B0.5, B0.6, B0.8, B1, B1.5, B2, B2.5, B3 |
| Embodiment 36 | B0.8, B1, B1.5, B2, B2.5, B3, B3.5, B4 |
| Embodiment 37 | C0.1, C0.3, C0.5, C0.6, C0.8, C1, C1.5, C2 |
| Embodiment 38 | C0.5, C0.6, C0.8, C1, C1.5, C2, C2.5, C3 |
| Embodiment 39 | C0.8, C1, C1.5, C2, C2.5, C3, C3.5, C4 |
| Embodiment 40 | D0.5, D0.6, D0.8, D1, D1.5, D2, D2.5, D3 |
| Embodiment 41 | D0.8, D1D1.5, D2, D2.5, D3, D3.5, D4 |

**Table 6 Combination relationship between the strength and the number of layers**

| | The strength of 6 layers | | | | | |
|---|---|---|---|---|---|---|
| Embodiment 1 | 300 | 320 | 400 | 450 | 580 | 700 |
| Embodiment 2 | 400 | 450 | 500 | 550 | 600 | 750 |
| Embodiment 3 | 500 | 540 | 620 | 760 | 850 | 900 |
| Embodiment 4 | 610 | 640 | 680 | 750 | 850 | 900 |
| Embodiment 5 | 700 | 750 | 850 | 950 | 1080 | 1200 |
| Embodiment 6 | 800 | 870 | 960 | 1100 | 1150 | 1300 |
| Embodiment 7 | 300 | 500 | 700 | 900 | 1100 | 1300 |

**Table 7 Combination relationship between the strength and the number of layers**

| Strength | The strength of 7 layers | | | | | | |
|---|---|---|---|---|---|---|---|
| Embodiment 17 | 350 | 400 | 480 | 550 | 660 | 800 | 900 |
| Embodiment 18 | 400 | 450 | 520 | 610 | 700 | 750 | 950 |
| Embodiment 19 | 520 | 580 | 620 | 760 | 840 | 900 | 1000 |
| Embodiment 20 | 610 | 640 | 680 | 750 | 850 | 940 | 980 |
| Embodiment 21 | 700 | 750 | 850 | 950 | 1080 | 1200 | 1250 |
| Embodiment 22 | 800 | 870 | 960 | 1100 | 1150 | 1250 | 1300 |

**Table 8 Combination relationship between the strength and the number of layers**

| | The strength of 8 layers | | | | | | | |
|---|---|---|---|---|---|---|---|---|
| Embodiment 30 | 350 | 400 | 450 | 550 | 660 | 750 | 850 | 900 |
| Embodiment 31 | 400 | 440 | 530 | 620 | 750 | 800 | 830 | 870 |
| Embodiment 32 | 520 | 580 | 620 | 760 | 840 | 900 | 1000 | 1100 |
| Embodiment 33 | 610 | 630 | 670 | 740 | 800 | 840 | 880 | 900 |
| Embodiment 34 | 750 | 810 | 850 | 950 | 1080 | 1200 | 1250 | 1300 |
| Embodiment 35 | 800 | 850 | 960 | 1050 | 1100 | 1140 | 1200 | 1300 |

The fixed strength of each layer in the same embodiment of the present application can correspond to the colors of different color systems, for example:

**Table 9 Combination of the strength, the number of layers and the color, and many embodiments can be listed, for example:**

| | 6 layers | | | | | |
|---|---|---|---|---|---|---|
| Embodiment 4 | 610 | 640 | 680 | 750 | 850 | 900 |
| Color 1 | C0.1 | C0.3 | C0.5 | C0.6 | C0.8 | C1 |
| Color 2 | A0.5 | A0.6 | A0.8 | A1 | A1.5 | A2 |
| Color 3 | C0.8 | C1 | C1.5 | C2 | C2.5 | C3 |
| Color 4 | B0.5 | B0.6 | B0.8 | B1 | B1.5 | B2 |
| Color 5 | C0.5 | C0.6 | C0.8 | C1 | C1.5 | C2 |
| Color 6 | D0.8 | D1 | D1.5 | D2 | D2.5 | D3 |

**Table 10 Combination of the strength, the number of layers and the color, and many embodiments can be listed, for example:**

| | 7 layers | | | | | | |
|---|---|---|---|---|---|---|---|
| Embodiment 20 | 610 | 640 | 680 | 750 | 850 | 940 | 980 |
| Color 7 | A0.3 | A0.5 | A0.6 | A0.8 | A1 | A1.5 | A2 |
| Color 8 | C0.5 | C0.8 | C1 | C1.5 | C2 | C2.5 | C3 |
| Color 9 | B0.3 | B0.5 | B0.6 | B0.8 | B1 | B1.5 | B2 |
| Color 10 | C0.3 | C0.5 | C0.6 | C0.8 | C1 | C1.5 | C2 |
| Color 11 | D0.5 | D0.8 | D1 | D1.5 | D2 | D2.5 | D3 |

**Table 11 Combination of the strength, the number of layers and the color, and many embodiments can be listed, for example:**

| | 8 layers | | | | | | | |
|---|---|---|---|---|---|---|---|---|
| Embodiment 32 | 520 | 580 | 620 | 760 | 840 | 900 | 1000 | 1100 |
| Color 12 | A0.1 | A0.3 | A0.5 | A0.6 | A0.8 | A1 | A1.5 | A2 |
| Color 13 | C0.3 | C0.5 | C0.8 | C1 | C1.5 | C2 | C2.5 | C3 |
| Color 14 | B0.1 | B0.3 | B0.5 | B0.6 | B0.8 | B1 | B1.5 | B2 |
| Color 15 | C0.1 | C0.3 | C0.5 | C0.6 | C0.8 | C1 | C1.5 | C2 |
| Color 16 | D0.3 | D0.5 | D0.8 | D1 | D1.5 | D2 | D2.5 | D3 |

**Table 12 Combination relationship between the number of layers and the weight percent contents of each layer of colored zirconia powder**

| | 6 layers (unit: w%) | | | | | |
|---|---|---|---|---|---|---|
| Embodiment | First layer | Second layer | Third layer | Fourth layer | Fifth layer | Sixth layer |
| Embodiment 1 | 15 | 17 | 18 | 17 | 15 | 18 |
| Embodiment 2 | 16 | 16 | 16 | 15 | 17 | 20 |
| Embodiment 3 | 17 | 14 | 16 | 15 | 13 | 25 |
| Embodiment 4 | 18 | 13 | 14 | 13 | 12 | 30 |
| Embodiment 5 | 19 | 12 | 11 | 11 | 12 | 35 |
| Embodiment 6 | 20 | 15 | 15 | 15 | 15 | 20 |
| Embodiment 7 | 22 | 7 | 8 | 7 | 8 | 48 |
| Embodiment 8 | 25 | 5 | 5 | 5 | 5 | 55 |

**Table 13 Combination relationship between the number of layers and the weight percent contents of each layer of colored zirconia powder**

| | 7 layers (unit: w%) | | | | | | |
|---|---|---|---|---|---|---|---|
| Embodiment | First layer | Second layer | Third layer | Fourth layer | Fifth layer | Sixth layer | Seventh layer |
| Embodiment 17 | 15 | 6 | 6 | 6 | 6 | 6 | 55 |
| Embodiment 18 | 16 | 8 | 6 | 7 | 8 | 10 | 45 |
| Embodiment 19 | 17 | 10 | 9 | 10 | 9 | 7 | 38 |
| Embodiment 20 | 18 | 13 | 11 | 10 | 12 | 10 | 26 |
| Embodiment 21 | 20 | 12 | 12 | 12 | 12 | 12 | 20 |
| Embodiment 22 | 21 | 12 | 12 | 12 | 13 | 12 | 18 |
| Embodiment 23 | 23 | 14 | 12 | 11 | 12 | 11 | 17 |
| Embodiment 24 | 25 | 15 | 13 | 11 | 10 | 11 | 15 |

**Table 14 Combination relationship between the number of layers and the weight percent contents of each layer of colored zirconia powder**

| | 8 layers (unit: w%) | | | | | | | |
|---|---|---|---|---|---|---|---|---|
| Embodiment | First layer | Second layer | Third layer | Fourth layer | Fifth layer | Sixth layer | Seventh layer | Eighth layer |
| Embodiment 30 | 15 | 11 | 11 | 11 | 11 | 11 | 10 | 20 |
| Embodiment 31 | 16 | 12 | 11 | 12 | 11 | 9 | 7 | 22 |
| Embodiment 32 | 17 | 9 | 10 | 9 | 11 | 9 | 10 | 25 |
| Embodiment 33 | 18 | 5 | 5 | 5 | 4 | 5 | 3 | 55 |
| Embodiment 34 | 20 | 7 | 7 | 7 | 7 | 7 | 7 | 38 |
| Embodiment 35 | 21 | 6 | 7 | 6 | 5 | 7 | 6 | 42 |
| Embodiment 36 | 23 | 7 | 5 | 4 | 5 | 6 | 5 | 45 |
| Embodiment 37 | 25 | 4 | 4 | 3 | 4 | 3 | 4 | 50 |

The parameters of dry pressing, isostatic cool pressing, pre-sintering and final sintering and other unnoted parameters in each embodiment of embodiments 4-41 in the present application are the same as those in embodiment 1.
The foregoing descriptions are merely preferred embodiments of the present application and illustration of the technical principles used. It should be understood by those skilled in the art that, the scope of the invention involved in the present application is not limited to the technical solutions formed by the specific combination of the above-mentioned technical features, and should also encompass other technical solutions formed by random combination of the above-mentioned technical features or equivalent features thereof without departing from the inventive concept, e.g. the technical solutions formed by the mutual replacement of the above-mentioned features and technical features with similar functions disclosed in the present application (but not limited thereto).

## Claims

1. A preparation method of a dental zirconia restoration material with uniform transition of strength and color, comprising the following steps:
(1) pouring colored zirconia powder into a dry pressing mould in accordance with a sequence of the strength from high to low and the color from dark to light for each layer, and performing dry pressing;
(2) performing isostatic cool pressing after the dry pressing;
(3) performing pre-sintering after the isostatic cool pressing to obtain a greenware; and
(4) performing CAD/CAM cutting on the greenware, and finally performing final sintering to obtain the dental zirconia restoration material.

2. The preparation method of claim 1, wherein the dental zirconia restoration material comprises 6-8 ceramic layers.

3. The preparation method of claim 2, wherein when a first ceramic layer is a cut end, the strength of the colored zirconia powder of the first ceramic layer is 300-800 MPa; the difference of the strength of the colored zirconia powder of two adjacent ceramic layers is 20-200 MPa; and the strength of the dental zirconia restoration material gradually increases from the cut end to the neck, and the color gradually becomes darker from the cut end to the neck.

4. The preparation method of claim 3, wherein the weight percent content of the colored zirconia powder of the first ceramic layer is 15-25%, the difference of the weight percent contents of the colored zirconia powder of two adjacent ceramic layers at the middle is 0±2%, and the weight percent content of the colored zirconia powder of the last ceramic layer is 18-55%.

5. The preparation method of claim 3, wherein the colored zirconia powder of each strength contains A, B, C and D color systems satisfying the colors of a VITA 16-color shade guide of the international colorimetric standard, wherein the color range of the A color system is A0.1-A4, the color range of the B color system is B0.1-B4, the color range of the C color system is C0.1-C4, and the color range of the D color system is D0.1-D4; and after the color system of one ceramic layer is determined, the color system is selected for the rest ceramic layers, and the rule is followed that the color gradually becomes darker from the cut end to the neck.

6. The preparation method of any one of claims 1-5, wherein the dry pressing pressure is 3-15 MPa, and the dry pressing time is 5-30s.

7. The preparation method of claim 6, wherein the isostatic cool pressing pressure is 80-270 MPa, and the pressure is maintained for 60-200s.

8. The preparation method of claim 7, wherein the pre-sintering temperature is 800-1100 °C, and the pre-sintering time is 90-180 min.

9. The preparation method of claim 8, wherein the final sintering temperature is 1450-1550 °C, and the final sintering time is 60-180 min.

10. A dental zirconia restoration material with uniform transition of strength and color prepared by the preparation method of any one of claims 1-9.
